(19) **Europäisches Patentamt / European Patent Office / Office européen des brevets**

(11) **EP 4 725 437 A1**

(12) **EUROPEAN PATENT APPLICATION**

(43) Date of publication:
**15.04.2026  Bulletin 2026/16**

(21) Application number: **24205429.4**

(22) Date of filing: **08.10.2024**

(51) International Patent Classification (IPC):
**A61B 18/22** (2006.01)

(52) Cooperative Patent Classification (CPC):
**A61B 18/22;** A61B 2018/00577; A61B 2018/00589;
A61B 2018/00642; A61B 2018/00672;
A61B 2018/00678; A61B 2018/00708;
A61B 2018/00809; A61B 2018/2261; A61N 5/0625;
A61N 5/067; A61N 2005/0626; A61N 2005/0659

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Clinical Laserthermia Systems AB**
**223 81 Lund (SE)**

(72) Inventors:
• **JOHANSSON, Jimmy**
**243 32 Höör (SE)**
• **PANTALEONE, Cristina**
**224 66 Lund (SE)**
• **KNAPPE, Verena**
**616 90 Åby (SE)**
• **AMBOLT, Petra**
**224 72 Lund (SE)**

(74) Representative: **KIPA AB**
**Drottninggatan 11**
**252 21 Helsingborg (SE)**

(54) **SYSTEM FOR THERMAL THERAPY**

(57) A computer implemented method or system for thermal therapy including obtaining at least a first magnetic resonance image showing a region to be heat treated and a heat providing area arrange in the region to be treated, wherein the heat providing area is associated with a heating device, defining a high temperature guard region encircling at least a portion of the heat providing area at a distance from the heat providing area, setting a maximum temperature for the high temperature guard region, and monitoring a temperature in the high temperature guard region based on voxels within the high temperature guard region during a heat treatment.

**Fig. 5A**

Description

BACKGROUND OF THE INVENTION

Field of the Invention

This invention pertains in general to the field of thermal therapy. More particularly the invention generally relates to a method and system for guarded thermal therapy for improved control and patient safety.

Description of Prior Art

This section is intended to introduce the reader to various aspects of art that may be related to various aspects of the present disclosure, which are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

Thermal therapy today using laser technology is performed with a cooled optical fibre and discrete temperature measurement points. The measurement points could either be thermal sensors arranged in the tissue to be treated or outside of the tissue to be treated as in WO2014/177663 A1 or selected points on a temperature map from magnetic resonance images.

These systems have the disadvantages that heating of the tissue to be treated can be slow due to the cooling of the optical fiber and that tissue closes to the probe may not be killed, again, due to the cooling of the cooled fiber. The accuracy of the thermal therapy is therefore not very precise, which may affect the treatment and the patient's safety due to unpredictable tissue damage and lesions sizes. It may also affect what type of organs can be treated using laser based thermal therapy.

Hence, notwithstanding the efforts in the prior art, there remains a need for further improvements to provide improved control of the thermal therapy. Improved control of the thermal therapy may provide for increased efficiency and improved patient's safety. Further, an improved control may reduce the risk gas being obtained in the tissue causing bubbling, charring/-carbonisation of tissue, and vapouring of the tissue during heat treatment of a tissue site. This may then provide that more types of organs can be treated using thermal therapy. Improved accuracy and control may provide optimization of an immune response, such as an anti-tumour immunologic effect, which may contribute to selective tissue damage and relatively small release of growth factors. By obtaining an immune response, the treated tissue will not only be destroyed by the provided heat, but the immune effect may destroy remaining tumour, locally or at distant sites, including lymph node This may give the possibility to use chemotherapy in a more efficient way since chemotherapy can be started before or at the time of local therapy.

Summary of the Invention

Accordingly, embodiments of the present disclosure preferably seek to mitigate, alleviate or eliminate one or more deficiencies, disadvantages or issues in the art, such as the above-identified, singly or in any combination by providing a method, such as a computer implemented method, a software and/or a system according to the appended patent claims for providing thermal therapy.

According to one aspect of the disclosure, a method for thermal therapy, such as computer implemented method or a computer software, includes:

   obtaining at least a first magnetic resonance image showing a region to be heat treated and a heat providing area arranged in the region to be treated, wherein the heat providing area is associated with a heating device.
   converting the magnetic resonance image into a heat map made of voxels;
   prompting a user to define a high temperature guard region encircling at least a portion of the heat providing area at a distance from the heat providing area;
   setting a maximum temperature for the high temperature guard region; and
   monitoring a temperature in the high temperature guard region based on voxels within the high temperature guard region during a heat treatment.

In some examples, the method may include switching off the heating device when the temperature reaches the maximum temperature. In some other examples, the method may include reducing the power of the heating device when the temperature reaches the maximum temperature. Reaches may in this disclosure include that the measured temperature is about to reach the maximum temperature, such as being close to the maximum temperature.

In some examples of the disclosure, the heating device may be a laser based heat probe and the heat providing area is a light emitting area of the heat probe.

In some examples, the heating device may be a High intensity focused ultrasound (HIFU) having a focus defined as the heat providing area.

In some examples, the method may include receiving information from an interface device about the heating device connected and setting the maximum temperature based on the heating device connected.

In some examples, the method may include receiving information from an interface device about the heating device connected and setting a max power and a recommended power based on the heating device connected.

In some examples, the maximum temperature may be reached when at least one voxel, or a plurality of voxels, such as a defined number of voxels, inside the high temperature guard region is determined at the maximum temperature.

In some examples, voxels within the high temperature guard region having a deviating temperature from neighboring voxels may be discarded.

In some examples, a region of interest covering the region to be heat treated may be defined for monitoring a thermal damage .

In some examples, the region of interest is the same as the high temperature guard region.

In some examples, the thermal damage is based on thermal dose calculation, such as a thermal damage estimate, based on temperature and exposure time .

In some examples, the region of interest may be divided into at least two different thermal damage thresholds (TDT) to display to display different levels of thermal damage.

In some examples, the method may include stop heat emission when an achieved thermal damage has been obtained based on a target damage and/or a target treatment time reached.

In some examples, a temperature monitoring guard may be defined outside or inside the region to be treated, for feedback control of the thermal treatment. The feedback control may include:

- warming time: wait until the temperature has reached a target temperature at the temperature monitoring guard;
- regulating the heating device based on the temperature measured by the temperature monitoring guard.

In some examples, a body temperature may be estimated at a body temperature estimation area not affected by the thermal treatment and the heat map is corrected based on any changes to the body temperature estimation area.

In some examples, the method may include initiating a test dose at a power lower than the power for thermal treatment for a period, such as a predefined period of time, of time to confirm a position of the heat providing area and/or confirming a position of the at least a first magnetic resonance image, such as a thermometry plane, in relation to the heating provided area.

In a second aspect of the disclosure, a system for thermal therapy is disclosed. The system may include:

a heating device comprising a heat providing area: and
a control unit configured to:
obtaining at least a first magnetic resonance image showing a region to be heat treated and the heat providing area arrange in the region to be treated,

converting the magnetic resonance image into a heat map made of voxels;

prompting a user to define a high temperature guard region encircling at least a portion of the heat providing area at a distance from the heat providing area;
setting a maximum temperature for the high temperature guard region; and
monitoring a temperature in the high temperature guard region based on voxels within the high temperature guard region during a heat treatment.

The describe invention may provide improved control of thermal therapy including, increased efficiency and improved patient's safety. The invention may also provide reduction of risk of gas being obtained in the tissue causing bubbling, charring/carbonisation of tissue, and/or vapouring of the tissue during heat treatment of a tissue site.

Improved control of the thermal therapy may also provide better temperature control of the treatment lesion or tissue to be treated so that cooled fibers may not be required which may provide faster and larger ablations or faster treatments and larger treatment sites.

Improved control of the thermal therapy may also provide the opportunity for more types of organs to be treated using thermal therapy.

Further, improved control of the thermal therapy may also provide optimization of an immune response, such as an anti-tumour immunologic effect, which may contribute to selective tissue damage and relatively small release of growth factors. By obtaining an immune response, the treated tissue may not only be destroyed by the provided heat, but the immune effect may destroy remaining tumour, locally or at distant sites, including lymph node This may give the possibility to use chemotherapy in a more efficient way since chemotherapy can be started before or at the time of local therapy.

It should be emphasized that the term "including/having" when used in this disclosure is taken to specify the presence of stated features, integers, steps or components but does not preclude the presence or addition of one or more other features, integers, steps, components or groups thereof.

## BRIEF DESCRIPTION OF THE DRAWINGS

These and other aspects, features and advantages of which embodiments of the invention are capable of will be apparent and elucidated from the following description of embodiments of the present disclosure, reference being made to the accompanying drawings, in which:

Figs. 1A and 1B are illustrating schematic examples of optical waveguides;
Fig. 2 is illustrating a capillary to be used to protect the emitting area of an optical waveguide;
Fig. 3 is illustrating a schematic treatment system according to the disclosure;

Figs. 4A to 4C are illustrating various schematic examples of high temperature guards;

Fig. 5A is illustrating a region of interest for monitoring a Thermal Dose Threshold;

Fig. 5B is illustrating a temperature monitoring guard defined as a line outside the region to be treated for feedback control of the thermal treatment;

Fig. 5C is illustrating a temperature monitoring guard arranged as a line encircling tissue to be treated;

Fig. 5D is illustrating an example where both a temperature monitoring guard and a region of interest is used for controlling a treatment;

Fig. 6 is illustrating an example of a test dose;

Fig. 7 is illustrating a schematic flow-chart of a treatment protocol for manual use;

Fig. 8 is illustrating a schematic flow-chart of a treatment protocol for feedback control; and

Fig. 9 is illustrating a schematic flow-chart of a method for thermal therapy.

## DESCRIPTION OF THE PREFERRED EMBODIMENTS

Specific examples will now be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

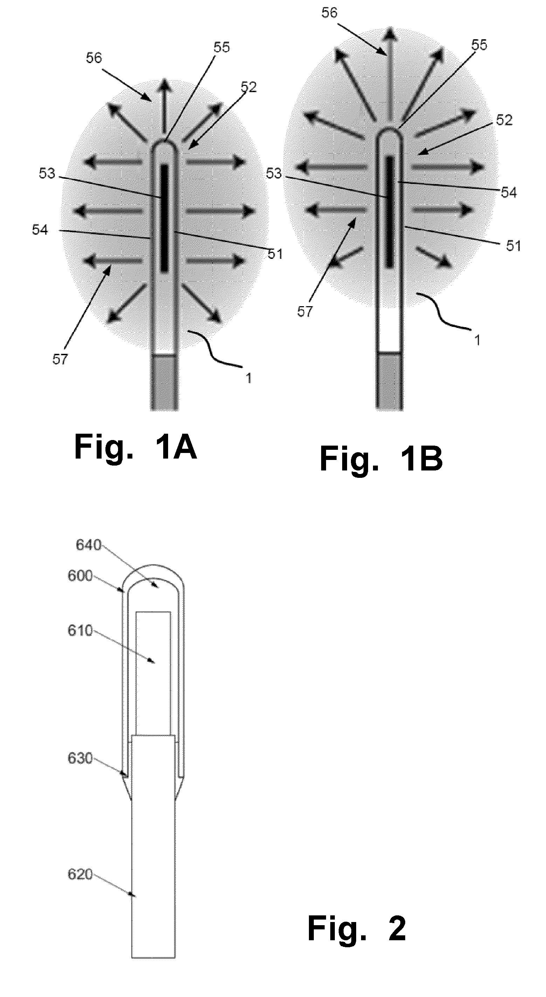

Fig. 1A and 1B are illustrating two schematic examples of optical waveguides 1, such as optical fibres. The optical waveguides 1 are adapted to be used for performing thermotherapy on at least a portion of a tissue site. Thermotherapy could here be irreversibly tissue damages for treatment purpose. The damage may be obtained by ablation through removing tissue by vaporisation, such as evaporation, or sublimation. Another example is to achieve coagulative temperatures without monitoring the progress of necrotised tissue over the time of the treatment, such as focal laser ablation (FLA), sometimes also referred to as laser-induced interstitial thermotherapy (LITT). During FLA or LITT the temperature may be monitored using MRI. The treatment may also be improved by obtaining an anti-tumour effect, such as an immunologic effect. The anti-tumour effect may be a local, distant or combined local/distant effect following local tumour destruction. The anti-tumour effect is triggered by antigens and may destroy any part left of a treated tumour but may also destroy other untreated tumours in the patient. Thus, the effect may be seen as a "vaccine" against a tumour (abscopal effect). The antigens are a result of a treatment causing cell death but without coagulating/denaturation of tumour antigens.

Each of the optical waveguides 1 in Fig. 1A and 1B includes a light emitting area 51 arranged at a distal portion 52. The distal portion 52 is partially a diffusor. The diffusor 52 may be made from a structured writing. The structured writing may be micro-modifications, and the micro-modifications are burnt into a core and/or cladding and/or buffer of the optical waveguide 1. The distal portion 52 of the waveguide 1 has an optical axis, and the micro-modifications are arranged along the optical axis so that light can be emitted from a lateral surface 54 and, in some examples, from an end surface 55 of the distal portion 52. Providing emission from an end surface 55 is a characteristic that may make optical waveguide 1 particularly suitable to be used in a heat device for treatment sites where the mechanical penetration depth has to be shallower than normal.

Another examples of applications for which the optical waveguide 1 may be used is in the field of neurosurgical applications such treatment of tissue where the tissue is brain tissue, for treatment of a neurological disease, such as treatment of tumors, brain, radiation necrosis, neurological functional disorders, Parkinson's disease or epilepsy centers in close proximity of eloquent structures, where a mechanical damage or an uncontrolled thermal spread may lead to functional or sensory impairment or even death, or treatment of suicidal tendencies, or other treatment sites such as urology applications such as treatment of tumorous tissue adjacent to the prostate capsule, where a perforation of the capsule may lead to fistula formation and potentially sepsis or nerve damage. In these fields the feature of providing emission from an end surface 55 of waveguide 1 may be particular useful. A treatment using waveguide 1 is particularly effective if combined with non-invasive real time temperature monitoring using, for example MR thermometry.

Controlling the amount of irradiation in a forward direction from an end surface 55 and irradiation in a radial direction from a lateral surface 54 provides the opportunity to adapt the diffusor to the treatment which contributes to the safety and effectiveness of the treatment especially in those cases in which the placement of the targeted area is close to anatomical structures that cannot be subject to damage otherwise causing severe adverse effects. In particular, a waveguide 1 of the disclosure may improve to the safety and effectiveness of the treatment by enabling the treatment as close as possible to sensitive anatomical structures without the need to mechanically insert the probe as deep into tissue as would be necessary with a standard forward irradiation profile. The risk of mechanical damage to these structures can thereby be minimised without compromising the size of treated tissue close to these sensitive structures. This result is achieved because the design allows a controlled deposit of energy beyond the tip of the heating device. The named deposit of energy can be tailored to a given application by varying the density and distribution of the micro modifications within the core.

For treatment of brain tissue, such as neurological disease, a suitable emittance of diffused light through the end surface 55 the diffusing optical waveguide end is about 5% to 25%, such as 5% to 15%, such as 8% to 12%, such as 10% to 25%, such as 15% to 25%, such as 20% to 40 25% of the total light being emitted from the diffusing optical waveguide tip or light emitting area 51. In other applications could a suitable emittance of diffused light through the end surface 55 of the diffusing optical waveguide be about 40% to 50%.

To control the light profile emitted for the optical waveguide tip or light emitting area 51, both from the lateral surface 54 and the end surface 55, the micro-modifications are varied along the optical axis. For example, the density of the micro-modifications may increase towards the light emitting end surface 55 to compensate for power losses in the light when propagated along the optical axis. This may achieve a flat top profile along the radial emission 57. The density of the micro-structures may vary by changing the distance between the micro-modifications along the optical axis. The density may increase by shortening the distance between the micro-modifications in the direction of the optical axis. The difference in density over the length of the diffuser 53 may be fairly large and go from 5 to 60 U/mm or from 5 to 40U/mm, where U is the number of micro-modifications. Other ranges of density may be required since the range may depend on the length of the diffuser. A shorter diffuser 53 may require a smaller range than a longer diffuser 53 to compensate for the loss in light power closer to the light emitting end surface 55 of the optical waveguide 1 due to the light propagating through the diffusor 53. Depending on the wanted light profile of the diffuser 53, the variation of the micro-modifications may not only involve an increase of the density towards the light emitting end surface 55 but could also be that there are sections of increased and decreased density spread out along the optical axis. The diffusor 53 may have different lengths depending on the application, but common lengths are between 5 to 30mm, such as 10 to 30mm, such as 15mm, such as 20mm such as 25mm. Additionally or alternatively to change the density of the micro-modifications may be to change the scattering strength by changing the scattering properties.

Fig. 2 is illustrating a capillary to be used to protect the emitting area of an optical waveguide, in this example, an optical fiber. The emitting area of the optical waveguide is a diffuser. The diffuser may be a diffuser as schematically illustrated in Figs. 1A and 1B. The optical waveguide, which may be an optical fiber, may be made of silica or a plastic, or may be any other suitable type of optical waveguide. The optical waveguide may also be a polymer coated waveguide.

Fig 2 illustrates a capillary 600 covering an end of a waveguide 610 which may include a diffuser section. The waveguide 610 and the diffuser section may be any type of waveguide and diffuser section described herein.

The diffuser section of waveguide 610 comprises of a waveguide core and may also in some examples include at least one cladding. The diffuser section may also include at least one jacket, coating and/or buffer. The tip of the distal end of waveguide 610 may be in some examples flat as illustrated in Fig. 2 or conical. The capillary 600 may be made from glass or plastic. The capillary may be bonded 630 to the jacket 620 and/or buffer of the waveguide either by fusing or by using an adhesive, and/or by shrink tubing. The space 640 around the bare end fiber 610 may be filled with air. This design of the capillary increases the waveguide's mechanical stability and its resistance to high temperatures. The heating device itself may comprise the jacket of which the optical fiber is made from, for example acrylate, then a layer of, for example, resin and an outer layer made of, for example, PBT, ETFE or PTFE.

When using laser light to heat and ablate tissue most diffuser fibers use a cooling system with the dual purpose of i) protecting the integrity of the device from high temperatures in the internal structures and ii) avoid carbonisation in tissue due to high temperatures in the interface between the heating device and treated tissue. If a cooling system is not used, the low temperature resistance in most diffuser designs will restrict the maximum laser power and thereby only small ablation sizes can be achieved. When controlling the laser light power there are some clear disadvantages to using cooling, e.g. increasing the treatment time and creating a risk of leaving viable tissue close to the heating device. It is also more difficult to control a treatment when energy is both deposited, laser light, and removed, cooling media. Using a cooling media also introduces a patient risk if supply of cooling media is interrupted which will destroy the heating device's internal structure leading to uncontrolled emission of laser power in tissue as well as leakage of cooling media into tissue. In a neuro application both these events may be catastrophic for the patient and in other applications this may lead to unintentional tissue destruction.

To achieve a safe treatment, using a non-cooled heating probe as a heating device, without the risk of structural damage to the probe, the materials in the probe must withstand high temperatures well over 100 $\underline{o}$C. Further to avoid tissue carbonisation at high laser power levels the energy density must be restricted. The current invention overcomes these limitations by using only high temperature resistant materials (protection against structural damage) and by using a capillary that covers the internal structure of the diffuser element. The capillary protects the internal structure from mechanical damage as well as lowers the energy density in the interface between the probe and tissue. For the same laser power, the energy density decreases as the square of the capillary radius. The combination of micro modification in a silica fiber and using a silica capillary, as in Fig. 2, makes it possible to design an un-cooled probe that can deliver high laser power into tissue.

Providing an un-cooled probe removes the need for a cooling system, reduces the complexity of the treatment procedure and of the devices involved in the treatment. When using a cooled solution, especially when the treatment is performed with MR guidance and/or MR thermometry, special installations may be required to be able to handle the cooling fluid. The non-cooled applicator does not require the use of fluid circulation devices and requires only a simplified setup if compared to a cooled solution, no tubing needs to be routed into the sterile field or in the MR room and no circulation system is required.

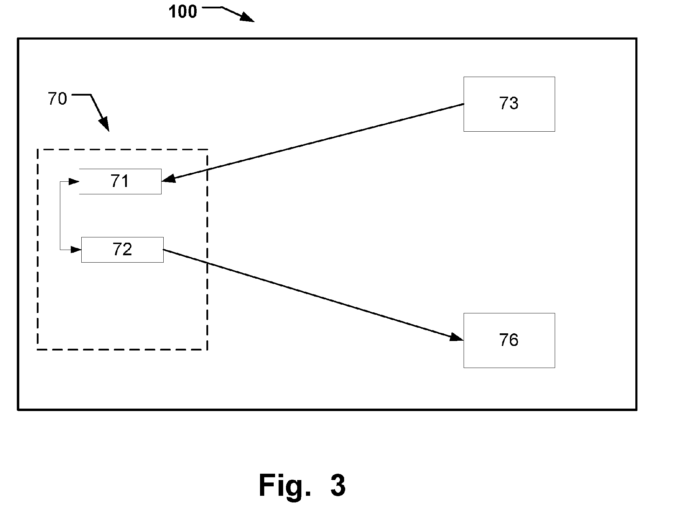

Fig. 3 is illustrating a schematic treatment system. The system is configured for controlling a thermotherapy of tissue. The thermotherapy is controlled by controlling a size of a treatment lesion covering at least a portion of tissue to be treated. Preferably the treatment lesion is sized to the whole tissue area to be treated by measuring element used for controlling the treatment outside the tissue area. The treatment as well as the size of the treatment lesion is controlled by monitoring the temperature of the tissue using MR thermometry. The temperature can either be controlled close to the heat probe and/or at a distance about 0 to 5 mm, such as 2 to 5mm, outside the boundary of the tissue to be treated, i.e. 0 to 5 mm, such as 2 to 5mm, outside the treatment lesion. When monitoring the temperature outside of the treatment lesion, the size of the treatment lesion may be determined by the distance between the energy emitting part of the probe and the point in the tissue where the temperature is selected to reach a target temperature, such as a target temperature between 40 to 60°C. In particular, the disclosure relates to a device, system, and method for obtaining coagulative temperatures and accurately monitoring the progression of necrotised tissue over time and/or obtaining an anti-tumour immunologic response by thermotherapy of at least a portion of a tissue site, such as a tumour.

Additionally and/or alternatively, in some examples, the treatment is controlled by monitoring the damage which may be calculated based on data from MR thermometry and time. This may be combined with a high temperature guard arranged close to the heat probe, within or at the boarder of the lesion.

For the present system, the modality used for monitoring the temperature may be the same modality used for investigating the treatment site and the surrounding tissue for establishing a size and shape of the treatment site. Thus, may be done using ultrasound, MRI or other suitable imaging modalities.

The heating probe used for obtaining the treatment lesion may be either based on a light guide as described in relation to Figs, 1A, 1B and 2, or the heating device may be a High intensity focused ultrasound (HIFU) having a focus defined as a heat providing area.

In an example according to Fig. 3, a schematic illustration over an exemplary system 100 for thermotherapy of tissue is illustrated. System 100 is especially developed for controlling the thermos therapy by controlling and/or monitoring the degree of the damage has occurred to the tissue to be treated, such as the size of a lesion is covering at least a portion of a treatment site. While controlling and/or monitoring the damage of the tissue, the temperature may also be monitored and used for controlling the treatment, such as used as a guard to avoid overheating the tissue to be treated and/or overheating healthy tissue outside of the lesion. The system 100 may in some examples be used for controlling immunostimulating laser thermotherapy.

System 100 comprises a control unit 70 which includes a temperature reading unit 71 configures to receive temperature data from a temperature measuring unit 73. Control unit 70 may be a computer, a microprocessor or an electronic circuit for converting an input signal to an output signal. Control unit 70 may for example be performed using a feed-back loop.

The temperature measuring unit 73 may be Magnetic Resonance Imaging for obtaining a 2D or 3D temperature maps of the treatment area. Various guards or regions of interest, as herein described, may be defined by determining a point, a line and/or an area in the temperature map. For example, may a temperature monitoring guard be defined at a distance, such as at or outside a border of tissue to be treated, such as a tumour border, from the heat providing area, such as a light emitting area. This region may define a treatment lesion and temperature in this region may be used to control the heat source in order to maintain the temperature at a predetermined value. Other regions in the 2D or 3D temperature map of the MR Image may also be defined as an automatic safety function shutting down the heat source if a threshold temperature is reached, such as a high temperature guard close to the heat providing area, such as encircling at least a portion of the heat providing area. Additionally and/or alternatively, a region of interest for monitoring a Thermal Dose Threshold may be defined.

Close to the tip of the heating device 76 is a heat providing area, such as a light emitting area for emitting light from the optical fiber, for heating a treatment lesion. Energy, in the example light of a certain power, is emitted from the heat providing area of the heating device 76, in operation thereof, for the heating of the lesion. The heating device 76 is preferably in apposition with the treatment lesion, or at least positioned at the treatment lesion. The treatment lesion may cover at least a portion of a site to be treated. The covered portion of a treatment site has a boundary adjacent healthy tissue surrounding the site. Additionally, the heating device 76 may emit diffused light from the heat providing area. The diffused light may be provided by fitting a distal end of the optical fiber with a light diffusor.

In the illustrated example only one heating device 76 is shown but depending on the size and shape of the treatment site to be treated more than one heating device 76 may be used.

The measured temperatures from the provided temperature measuring unit 73 may be used as input to the control unit 71 for adjusting the power output of the laser generator 72 by adjusting the power of a laser source thereof, for example by a feedback system.

If the temperature at any of the temperature measure areas, lines and/or points exceeds a predetermined temperature value, the control unit 71 may decrease the power output of the laser generator 72. Alternatively, control unit 71 may inactivate the laser generator 72. If the temperature at any of the temperature measure areas and/or points becomes less than a predetermined temperature value, the control unit 71 may increase the power output of the laser generator 72. Alternatively, the control unit 71 may activate the laser generator 72, if it previously has been inactivated. The predetermined temperature may be a single maximum or target value. Alternatively, the predetermined temperature may be a range having an upper and a lower threshold temperature.

The adjustment of the output power may be done automatically by control unit 71. This could for example be done by a control algorithm implemented in the software of control unit 71. Alternatively and/or additionally, the control unit 71 may provide an alarm, such as to alert a medical practitioner to manually set and/or adjust the power output of the laser generator 72.

The wavelength may be any wavelength as long as there is a suitable absorption in the irradiated tissue to generate heat. Preferably, the wavelength should have a high penetration close to the heating device 76, i.e. low absorption. A too high absorption of energy may increase the delivered heat and temperature very rapidly around the heating device 76, thus it may not provide enough energy at the boundary of the at least portion of a treatment site covered by the treatment lesion. For example, a wavelength with suitable absorption may be found in the visual or near infrared wavelength region, such as in the region of 700 to 1300nm, such as 900 to 11 00nm such as 980nm, such as 1064 nm. Also, as known to the skilled person, scattering of the light will have a role in how the energy is transferred within the tissue.

In some examples, at least an area, line and/or points may be defined as a temperature monitoring guard for monitoring the temperature either at the boundary of the at least portion of a tissue site to be treated or at a distance from the boundary of the treatment lesion may be done by a temperature measuring unit 73. The temperature monitoring guard can be defined to be at least an area or points 0-1 0mm, such as 2-7 mm, such as between 2 to 5 mm, outside an established boundary of a treatment lesion. The choice of distance depends on the characteristics of the treatment site and the surrounding tissue. The target temperature may be in the range of 40 to 60°C, such as, such as 40 to 55°C, such as 44 to 48°C for obtaining an optimized treatment.

In some examples, the target temperatures may be kept stable for a treatment time between 20 to 60 minutes. Preferably the treatment time may be about 30 minutes. Before starting the treatment time, the target temperatures need to be reached. During this warm-up stage the laser output is adjusted until the right temperature is obtained either at the boundary of the at least portion of tissue to be treated or at a distance outside a boundary. The time to target may take between 5 to 15 minutes, such as between 5 to 10 min. Time to target could also be less than 1 min such as 30 sec. Time to target depends on the same parameters as previously mentioned, for example, maximum power output and optical coefficients of the different tissues and between different patients.

The heating device 76 may comprise a fiber having a light emitting area at the distal end, wherein the distal end is configured to be interstitially arranged in the tissue to be treated. In some examples, the heating device 76 has capillary arranged over the energy emitting area, such as the light emitting area. Wherein the capped end of the fibre is the distal end, and wherein the distal end is configured to be interstitially arranged in the tissue, such as the tumour. The capillary may, especially when a fiber is used as a heating device 76, improve the heat and mechanical stability of the heat probe. In a further example, the light probe is only a fiber having a light emitting area at the distal end, wherein the distal end is configured to be interstitially arranged in the tissue to be treated.

Additionally, in some examples, the system 100 may include further temperature measuring area and/or points arranged outside of the treatment lesion. These external temperature measuring points may be positioned, for example, next to sensitive anatomical structures that need to be protected from high temperatures. These external temperature measuring points may therefore serve as guards shutting off the heat source at a predetermined level to avoid damage to the sensitive structure.

The control unit 70 further comprises a power controlling unit 72, such as an energy source, for controlling the energy emitted by the heating device 76. The energy causing the heating of the tissue may be emitted using for example ultrasound technology or laser technology. Laser technology may be preferred as it has been shown to improve the control and heating of the tissue to be treated thereby improving the accuracy of optimizing the treatment lesion.

In this disclosure, a control unit, such as a computer is used for monitoring and/or controlling the treatment. The control unit, such as a computer, may be used for signal processing and evaluation of the measured data. All determinations or calculations described herein may be performed by a control unit or a data processing device (not illustrated).

The control unit or a data processing device may be implemented by special-purpose software (or firmware) run on one or more general-purpose or special-purpose computing devices. In this context, it is to be understood that each "element" or "means" of such a computing device refers to a conceptual equivalent of a method step; there is not always a one-to-one correspondence between elements/means and particular pieces of hardware or software routines. One piece of hardware sometimes comprises different means/elements. For example, a processing unit serves as one element/means when executing one instruction but serves as another element/means when executing another instruction. In addition, one element/means may be implemented by one instruction in some cases, but by a plurality of instructions in some other cases. Such a software-controlled computing device may include one or more processing units, e.g., a CPU ("Central Processing Unit"), a DSP ("Digital Signal Processor"), an ASIC ("Application-Specific Integrated Circuit"), discrete analogue and/or digital components, or some other programmable logical device, such as an FPGA ("Field Programmable Gate Array"). The data processing device may further include a system memory and a system bus that couples various system components including the system memory to the processing unit. The system bus may be any of several types of bus structures including a memory bus or memory controller, a peripheral bus, and a local bus using any of a variety of bus architectures. The system memory may include computer storage media in the form of volatile and/or non-volatile memory such as read only memory (ROM), random access memory (RAM) and flash memory. The special purpose software may be stored in the system memory, or on other removable/non-removable volatile/non-volatile computer storage media which is included in or accessible to the computing device, such as magnetic media, optical media, flash memory cards, digital tape, solid state RAM, solid state ROM, etc. The data processing device may include one or more communication interfaces, such as a serial interface, a parallel interface, a USB interface, a wireless interface, a network adapter, etc., as well as one or more data acquisition devices, such as an A/D converter.

The special-purpose software may be provided to the control unit or data processing device on any suitable computer readable medium, including a record medium and a read-only memory.

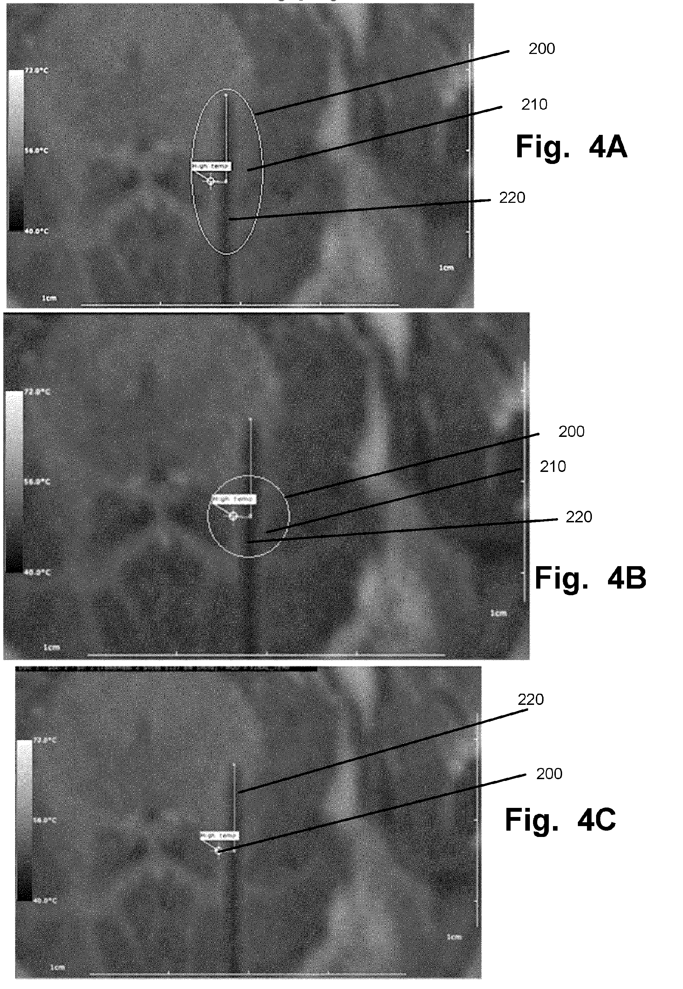

Figs. 4A to 4C illustrate various examples of high temperature guards 200 that limits the maximum temperature at a region of interest 210 close to a heat providing area 220 associated with a heating device. A high temperature guard 200 is defined to limit the risk of gas being obtained in the tissue causing bubbling, charring/carbonisation of tissue, and/or vapouring of the tissue during heat treatment of a tissue site. Obtaining gas or bubbling in the tissue during treatment may risk that the phase information in the MR images is lost. If the phase information is lost, the MRI temperature map will not work and the treatment will stop as it would otherwise mean that the treatment has to be performed without any temperature information and/or tissues damage estimate. A loss of phase information may also mean that the treatment may be performed with the wrong information, such as indicating a too high or low temperature and/or damage estimate.

Further, bubbles may cause disturbances in the images provided which may stay and affect a baseline for the temperature measurements.

A high temperature guard 200 may be set by obtaining at least a first magnetic resonance image showing a region to be heat treated and a heat providing area arrange in the region to be treated, wherein the heat providing area is associated with a heating device 220. The at least first magnetic resonance image is displayed on a display connected to a control unit which may be configured for controlling a power output of the heating device, such as a control unit as previously described herein.

The at least one magnetic resonance image is converted into a heat map made of voxels. The voxels may have a size of 0,8x0,8x3mm, which provides a compromise between signal and resolution. Temperature maps or heat maps may be calculated based on phase information from MR images acquired in near real-time during the treatment. The MR images may be acquired every 1 to 30 seconds, such as 1 to 7sec, such as 1 to 5sec. The control unit may be configured so that the heating for the treatment is switched off should an MR image be received outside a time interval, such as longer than 7 sec, such as longer than 5 sec. This is to make sure that the information provided to the clinician is accurate. The temperature calculation relies on a physical property of water molecules in the liquid state, namely that the water molecule's chemical shift varies linearly with temperature, regardless of its chemical environment, over a broad range of temperatures ($0°C < T < 100°C$) . This property is the basis of a temperature imaging technique called the Proton Resonance Frequency (PRF) shift method. The PRF shift may be imaged in near real time using fast imaging sequences and observing the phase change of the MR signal, which is directly proportional to the temperature change.

One method that may be used to implement the calculations using MR phase images to measure the PRF and to calculate temperature maps is described by Ishihara, Y. et al, "A precise and fast temperature mapping using water proton chemical shift", Magnetic Resonance in Medicine, 34(6), 814-823, which is hereby incorporated in its entirety. Advantages with this method is:

> It is tissue independent. The proportionality constant between the PRF shift and
> temperature change is the same for all water molecules regardless of tissue types.

The relationship between PRF shift and temperature is linear over the range of temperatures that is of interest in thermal ablation ($0°C < T < 100°C$).

Except for adipose tissue (the chemical shift of protons in fat does not change with temperature), the linear relationship between PRF and temperature is tissue typeindependent. The relation between temperature change and phase change in the MR images is very simply expressed by Equation 1:

$$\Delta T = \frac{\varphi(T) - \varphi(T_0)}{\alpha\,(\gamma B_0)\,TE} \qquad (1)$$

Where $\Delta T = T - T_0$ is the temperature variation, $\varphi$ is the voxel phase, $\gamma B_0$ is the magnetic field strength expressed in angular frequency unit, $TE$ is the pulse sequence Echo Time, and $\alpha$ is the PRF temperature coefficient, which, for non-fat tissue, has a value of $-0.0095 \pm 0.0005$ ppm/°C. In this equation, only the $B_0$ term changes from 7T to 0.5T, such as from 3.0T to 1.5T depending on the scanner being used to acquire images.

To avoid errors in the temperature maps due to phase drift, the MR images may need to be corrected. The correction may be made by defining at least one area of voxels at a distance from the treatment site. An area may be defined as a plurality of adjacent voxels. The number of voxels have to be large enough to obtain reliable temperature information, such as 10 voxels, such as 50 voxels. The distance should preferably be large enough to avoid the area is affected by the heating of the tissue. The area of voxels should preferably be in the same organ as the tissue site being treated. If a deviation from a baseline (body temperature) is observed in this area, the whole MR image may be corrected for the observed phase drift. For example, a body temperature may be estimated at a body temperature estimation area not affected by the thermal treatment and the heat map is corrected based on any changes of the body temperature estimation area.

The user is prompted to define a region 210 as a high temperature guard 200. The high temperature guard 200 may encircle at least a portion of the heat providing area 220 of a heating device at a distance from the heat providing area 220. Fig. 4A is illustrating a defined high temperature guard 200 encircling the whole heat providing area 220 of a heating device, Fig. 4B is illustrating a defined high temperature guard 200 encircling a portion of a heat providing area 220 of a heating device, and Fig. 4C is illustrating a defined high temperature guard 200 which is a point made of a plurality of voxels, or as a line along the heat providing area 220 of a heating device.

The high temperature guard 200 shall encompass a portion of tissue at a distance from the heat providing area 220. The distance has to be large enough to prevent issues with artefacts. For example, the distance has to be larger than 2 to 3 mm from the heat providing area 220. For Figs. 4A and 4B, all voxels inside the defined region 210 are used for monitoring the temperature. The high temperature guard 200 may be defined by a user, for example a user using an input device connected to the control unit to define the high temperature guard 200, such as using a drawing tool or entering digits defining the high temperature guard 200, or a combination thereof. Additionally and/or alternatively, the high temperature guard 200 may be defined automatically by the control unit, for example based on image analysis.

A maximum temperature is set for the high temperature guard region 210. The maximum temperature may be set automatically by the control unit receiving information of the type of heating device connected from an interface device. For example, the control unit may receive information of the type of heating device from an RFID tag, EEPROM or other wireless technology read by the interface device. The heating device information may be read by a laser unit to which the heating device is connected. In a further Example the maximum temperature may be set by the control unit based on information of the type of heating device connected and the type of tissue being treated. In an alternative, a recommendation for a maximum temperature may be provided by the control unit based on information of the type of heating device and/or tissue type to be treated. In a further alternative, the user can define a maximum temperature based on the type of heating device and/or tissue type to be treated. For example, the maximum temperature may be in the range of 70 to 100°C, such as 75 to 90°C, such as 85°C. Under most circumstances, a temperature under 100°C should be considered safe. Due to the update frequence of the temperature maps and, the temperature is normally set lower than 100°C, such as 75 to 90°C, since the temperature may continue to increase after the set temperature has been observed and the heating device has been switched off or the power to the heating device has been reduced.

Alternatively and/or additionally, a maximum and/or recommended power of the heating device may be set. The maximum and/or recommended power may be set automatically by the control unit receiving information of the type of heating device connected from an interface device. For example, the control unit may receive information of the type of heating device from an RFID tag, EEPROM or other wireless technology read by the interface device. The heating device information may be read by a laser unit to which the heating device is connected. In a further example, the maximum and/or recommended power of the heating device may be set by the control unit based on information of the type of heating device connected and the type of tissue being treated. In an alternative, a recommendation for a maximum and/or recommended power may be provided by the control unit based on information of the type of heating device and/or tissue type to be treated and/or voxel size and/or MR scanner parameters etc. some of this information may be provided to the control unit by the User. In a further alternative, the user can define a maximum and/or recommended power for the heating device based on the type of heating device and/or tissue type to be treated and/or voxel size and/or MR scanner parameters etc.

A temperature may be monitored in the high temperature guard region 210 based on voxels within the high temperature guard region 210 during a heat treatment. The maximum temperature may be considered reached when at least one voxel, such as a plurality of voxels, such as a group of voxels, inside the high temperature guard region 210 is determined at the maximum temperature. In some examples, when the maximum temperature is reached, the power to the heating device is switched off or reduced, such as, the laser unit connected to the heating device is switched off or an output power of the heating device is reduced by the laser unit controlled by the control unit.

Using a region encircling at least a portion of the heat providing area 220 of a heating device provides a higher accuracy of the temperature monitoring since it is not possible to know where the highest temperature will be measured in advance. All voxels inside the high temperature guard region 210 are used for monitoring the temperature. The voxels that do not have a sufficiently high signal-to-noise ratio are discarded and not used for the high temperature guard region 210. Discarded voxel may be removed or switched off. For example, voxels within the high temperature guard region 210 having a deviating temperature from neighboring voxels are discarded. Voxels having a temperature value that is either under and/or above a thresholds value before the treatment starts may be discarded, this may for example be voxels covering the heat providing area 210 of the heating device or voxels close to the heat providing area 210. This allows that unnecessary treatment interruptions due to noise can be avoided and reduce the risks of retreatment of a patient, which is not risk free.

Additionally and or alternatively, voxels not having a sufficiently high signal-to-noise ratio are discarded in the whole temperature map to reduce noise and improve the quality of the temperature map. An improved quality of the temperature map may improve other calculations and estimates based on the information from the temperature map. Noise may cause disturbances in the images provided which may also affect a baseline for the temperature measurements.

The heat map may be one or more in 2D-images. When more than one 2D map is used, different maps may show different planes to provide "3D" information. Multiple planes may also be combined to reconstruct a 3D view.

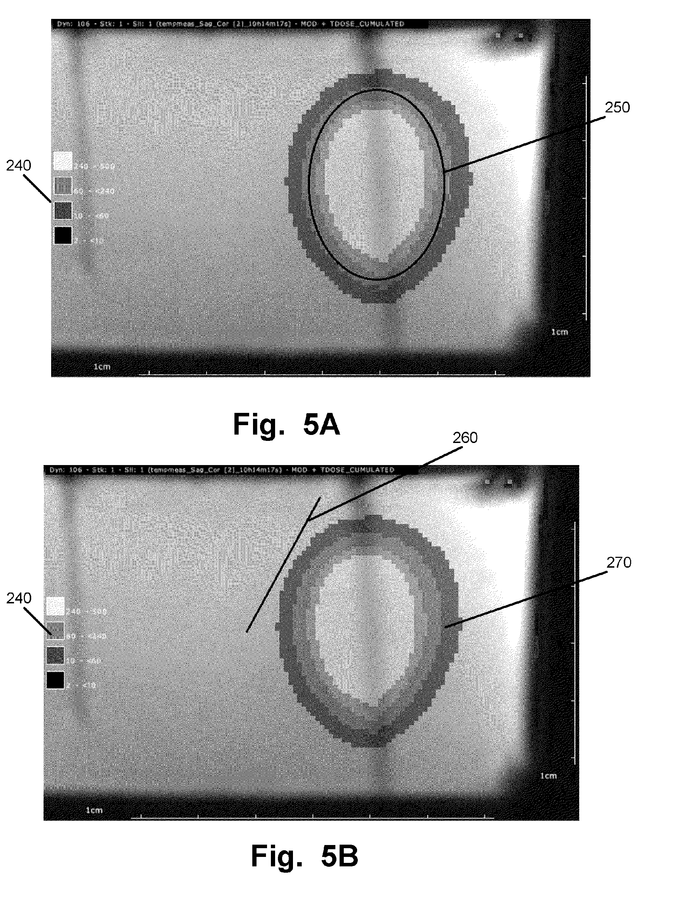

Figs. 5A to 5D are illustrating Thermal Damage Estimate (TDE) monitoring. The damage calculations may be a thermal dose calculation based on temperature and time. The Thermal Damage Estimation (TDE) may be based on Sapareto and Dewey's thermal Dose calculation, Sapareto, SA and Dewey, WC: "Thermal dose determination in cancer therapy", Int J Radiat Oncol Biol Phys., Vol. 10, pp. 787-800, 1984, which is hereby incorporated in its entirety. The method that may be used for calculating the thermal damage maps is known as Cumulative Equivalent Minutes at 43°C (CEM$_{43}$). The CEM$_{43}$ model is the *cumulative equivalent time* at the reference temperature of 43°C and is computed by using Equation 2:

$$CEM43 = \int_0^\tau R^{43-T} dt$$

$$(2)$$

where $T$ is temperature (°C), $t$ is time (minutes), $\tau$ is total time (minutes), and the weighting factor $R$ is equal to 0.5 for $T \geq 43$°C, and 0.25 for $T < 43$°C. Thermal damage estimate is independent of the MRI scanner magnetic field. It only depends on the temperature and the accumulated heating time.

Different levels of probability of thermal probability of thermal damage may be displayed as Thermal Dose Thresholds (TDT). In the examples illustrated in Figs. 5A to 5D four Thermal Dose Thresholds (TDT) levels 240 are displayed. The number of TDT levels may be more or fewer than this.

Usually, areas receiving a measured thermal dose will be depicted with different colors for different thermal doses as a cumulative effect on the tissue. The outer boundary for each dose level is known as Thermal Dose Threshold (TDT). In the gray scale images provided, the colours cannot be seen, however, the four unique Thermal Dose Thresholds (TDT) levels 240 may, for example, be displayed as:

**1. Green** Thermal Dose Threshold:

2-9CEM$_{43}$. Contained tissue has been exposed to the thermal equivalent of 43°C for at least 2 minutes duration. Tissue outside the green area has a high probability of receiving no thermal damage. Tissue in the Green area may experience thermal damage, but cell death is not guaranteed.

**2. Yellow** Thermal Dose Threshold:

10-59CEM$_{43}$. Contained tissue has been exposed to the thermal equivalent of 43°C for at least 10 minutes duration. Tissues with a yellow thermal dose are likely to experience irreversible cell damage.

**3. Orange** Thermal Dose Threshold:

60-239CEM$_{43}$. Contained tissue has been exposed to the thermal equivalent of 43°C for at least 60 minutes duration. All tissue within this boundary experiences cell death within ≤48 hours.

**4. Red** Thermal Dose Threshold:

The thermal equivalent of 43°C for ≥240 minutes duration. Irreversible damage has occurred.

The Thermal Dose Threshold may be provided as masks one or more in 2D-images. When more than one 2D-image is used, different images may show different angles to provide "3D" information of the thermal damage. Multiple planes may also be combined to reconstruct a 3D view of the thermal damage.

An alternative to using CEM43 for TDE is to use Arrhenius equations to obtain information when the treatment is completed.

In Fig. 5A, a region of interest 250 for monitoring a Thermal Dose Threshold (TDT) for treatment is illustrated. The region of interest 250 may be defined as an area. The region of interest 250 may covering the region to be heat treated. In some examples, the region of interest is the same as the high temperature guard region. When a certain level of tissue damage has been achieved within the area of the region of interest 250, the treatment goal may be considered as reached and the treatment is interrupted, for example by switching off or reducing the power to the heating device. The level of tissue damage can be set based on various factors, such as the type of tissue to be treated, the organ and/or the number of treatments needed.

In this way the heat treatment may not only be controlled by the temperature but also by the amount of damage that has been achieved to the tissue site to be treated. In some examples, the damage can be used as a feedback for controlling the treatment.

In Fig. 5B, a temperature monitoring guard 260 is defined as a line outside the region to be treated for feedback control of the thermal treatment. Voxels at the line and/or adjacent to the line may be used for measuring a temperature for the feedback control. Alternatively and/or additional, the temperature monitoring guard 260 may be a dot or an area, for example may an area be defined as a number of voxels encircled which may define an area to be used as a temperature monitoring guard 260. The guard 260 can be set either at a maximum temperature and/or as a maximum TDT. The Damage caused to the tissue to be treated is visualized as an overlaying mask 270. In some examples, the temperature monitoring guard 260 may be arranged inside the region to be treated.

The temperature monitoring guard 260, may be used to avoid damaging healthy tissue outside the tissue site to be treated. The temperature monitoring guard 260 may also be used to control the TDT by keeping the temperature at a predetermined level. When either a maximum temperature has been exceeded for the temperature monitoring guard 260 or a maximum TDT has been reached at the temperature monitoring guard 260, the treatment is, at least partially, interrupted, for example by switching off or reducing the power to the heating device. The interruption could be until the temperature has reduced to a level under the maximum temperature.

The temperature monitoring guard 260 may be used as a feedback control to the treatment, for example by performing the following steps:

- warming time: wait until the temperature has reached a target temperature at the temperature monitoring guard 260 and/or a damage threshold;
- regulating the heating device based on the temperature measured by the temperature monitoring guard 260;
- stop heat emission when achieved damage has been obtained based on target damage, target treatment time reached or interrupted by guard temperature/damage reached.

The temperature monitoring guard 260 may also be used to protect sensitive organs by arranging a temperature monitoring guard 260 close to, such as adjacent, an organ to tissue site to be protected. Additionally and/or alternatively, the temperature monitoring guard may use information about the tissue damage reached at the temperature monitoring guard to protect the sensitive organ. If a temperature and/or a tissue damage is reached, or is near to be reached, at the temperature monitoring guard that may affect the organ the heating device may be switched off or the power to the heating device may be reduced.

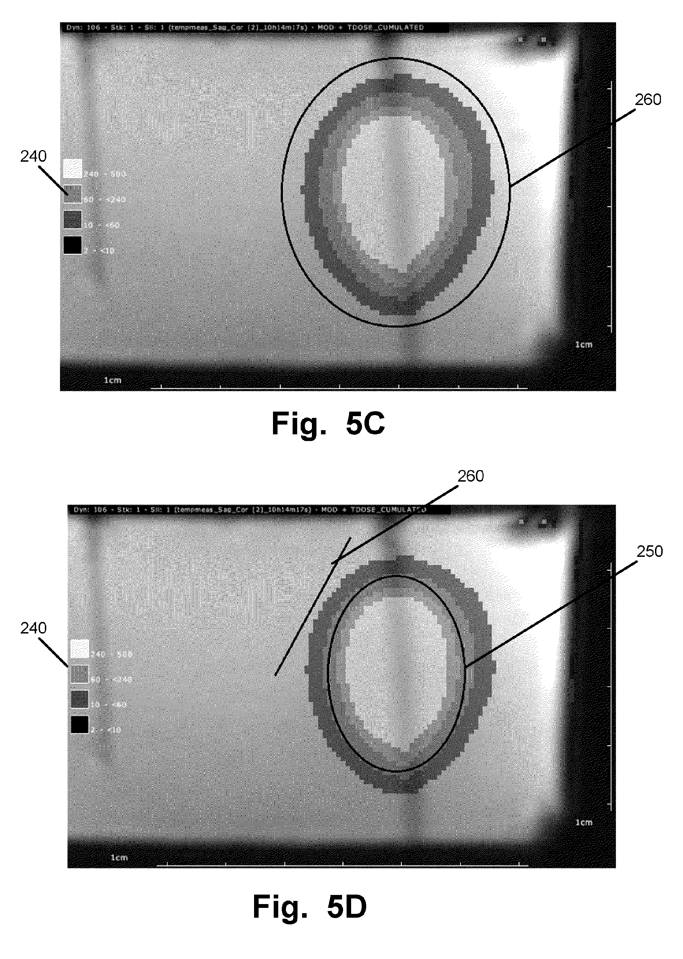

Fig. 5C defines an alternative to a temperature monitoring guard 260 arranged as a line outside the region to be treated. For example, the temperature monitoring guard 260 may be arranged to encircle the tissue to be treated. Voxels at the line and/or adjacent to the line may be used for measuring a temperature for feedback control.

Fig. 5D is illustrating an example where both a temperature monitoring guard 260 and a a region of interest 250 is used for controlling the treatment.

In some examples may the TDT be visualized differently based on the tissue type or the organ to be treated, such as the brain or the prostate. In the same way, the data for the treatment may be adjusted and handled differently depending on the organ to be treated. In some examples, different visualizations could be profiles, and the different visualization may be used depending on the treatments. For example, different algorithms may be used to estimate the damage depending on the treatment and/or the tissue and/or the organ to be treated.

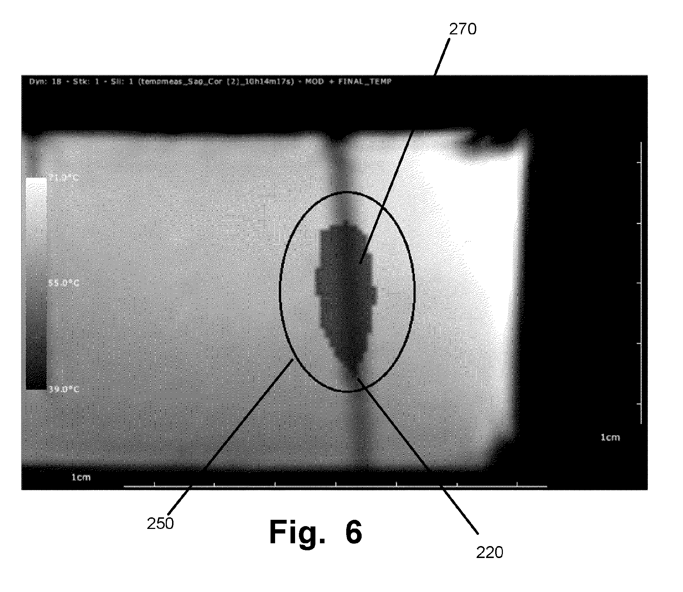

Fig. 6 is illustrating a test dose. A test dose is initially used at a power lower than the power for thermal treatment for a period of time to confirm a position of the heat providing area and/or confirming a position of the at least a first magnetic resonance image, such as a thermometry plane, in relation to the heating provided area. The power and/or the time is set automatically depending on the heating device attached to the system. The power and/or the time is set automatically to avoid or lower the risk of obtaining any damage to the tissue during the test dose. The time to run the test dose could be up to 30 sec The use of a test dose is in particular important when treating brain tissue, but could be useful for other organs as well.

In Fig. 6 the heat providing area is seen together with an overlay of a temperature map 270 showing that there is an increase of heat around a heat providing area 220 of the heating device. Further a temperature monitoring guard 260 or a high temperature guard 200 is also seen. The temperature monitoring guard 260 or a high temperature guard 200 may be used during the test dose to further limit the risk of any damage to the tissue during the test dose. Should the position of the heating device and/or a position of the at least a first magnetic resonance image, such as a thermometry plane, not be correct, the position of the heating device and/or the image plane, such as the thermometry, can be adjusted until it is correct.

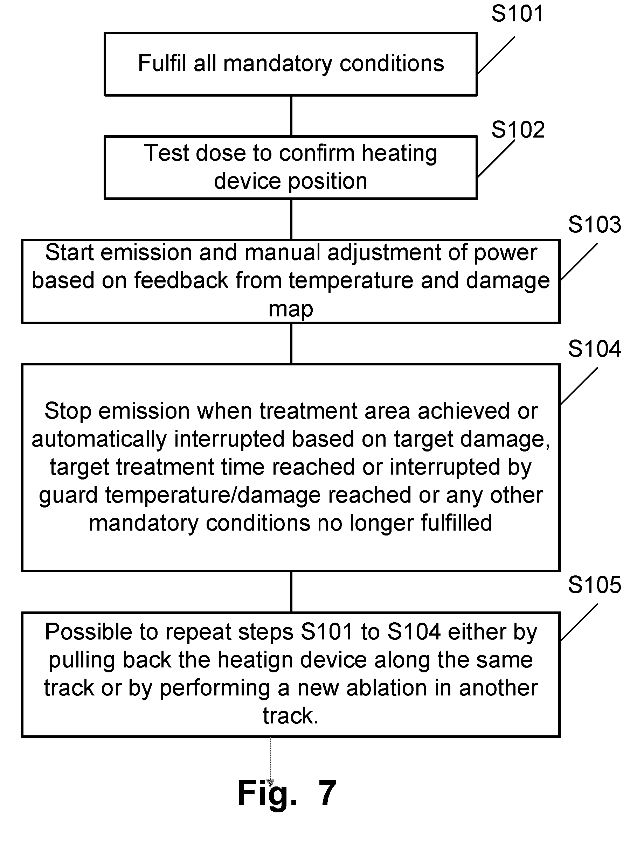

Fig. 7 is a schematic flow-chart of a treatment protocol for manual use. The treatment may for example be ablation. The method steps may include:

S101: Check if all mandatory conditions are fulfilled. The mandatory conditions have to be checked before starting to ensure correct setup before heat emission is started, such as before the laser is started. Should the mandatory conditions not be fulfilled, the control unit and/or software may not allow the start of the heating device, such as the laser. The method may include a self-check where the system and/or the software is configured to check that images are received, and that the temperature is correctly computed. This may also include a check to confirm that the heating device is correctly connected and what type of heating device is connected, such as reading information from the connected device, such as reading a RFID tag. Other conditions may be, that the laser unit is enabled, and a connection is verified. A loss in communication for a period of time, such as a period of 1.5sec to 7 sec, such as 1.5 sec, such as 5, sec, such as 7 sec, between the laser unit and the system or data incoherency may automatically stop the laser emission. In this way, the laser is turned off when the images are not updated and there is a risk that the user does not have near-real time information (loss of information and prolonged update rate can lead to unintentional tissue damage outside the treatment area which may be very bad when performing treatment of the brain and also in some other types of tissue.

Other mandatory conditions that may need to be fulfilled are baseline temperature is set and confirmed, such as core body temperature is confirmed; drift compensation: reference baseline is set and confirmed; that a high temperature guard is provided, that there is a communication with the MR scanner, the test dose is successful and confirmed etc.

S102: Test dose to confirm position of the heat providing area of the heating device, such as a laser-based heating device. This may be carried out by emitting a reduced power for a time, such as up to 30 sec. The power can be increased first after the test dose has been confirmed.

S103: Start emission from the heating device, such as start laser emission. The emission typically starts at reduced power and manual adjustment of power based on feedback from temperature and damage map.

S104: Stop laser emission when treatment area achieved or automatically interrupted based on target damage, target treatment time reached or interrupted by guard temperature/damage reached or any other mandatory conditions no longer fulfilled.

S105: Possible to repeat steps S101 to S104 either by pulling back the fiber along the same track or by performing a new ablation in another track. For pull-back, the heating device may be configured to include an introducer which can be left with the heating device in tissue during treatment. The introducer may be transparent to the emission, such as transparent to the laser wavelength used. During the pull-back the heating device, such as an optical fiber, can be pulled back within the introducer which is left in its position. This provides the advantage that the risk of losing the position during the manipulation of the heating device during the pull-back is decreased. The heating device may also be protected at the same time. Alternatively, the introducer may be pulled-back too, thereby allowing the distal end of the heating device, such as the heat providing area which may be a diffusing portion of an optical fiber, to be in direct contact with the tissue.

Additionally and/or alternatively, as described for pull-back, an introducer may be configured to be left with the heating device in the tissue even if no pull-back should be performed.

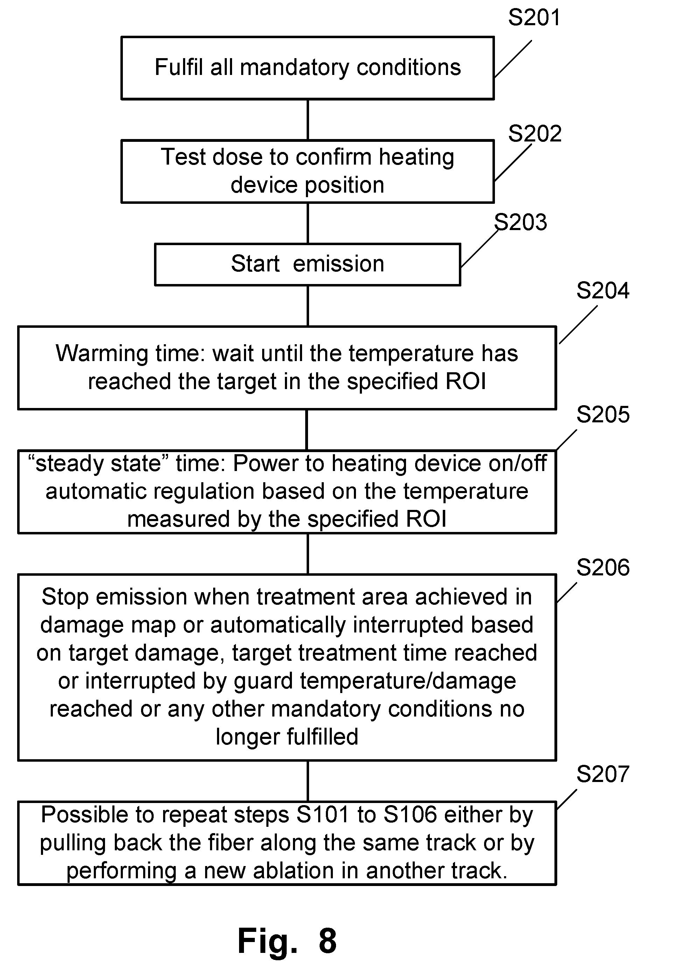

Fig. 8 is a schematic flow-chart of a treatment protocol for feedback control. The treatment may for example be ablation. The method steps may include:

S201: Fulfil all mandatory conditions. The mandatory conditions may have to be checked before starting to ensure correct setup before heat emission is started, such as before the laser is started. Should the mandatory conditions not be fulfilled, the control unit and/or software may not allow the start of the heating device, such as the laser. The method may include a self-check where the system and/or the software is configured to check that images are received, and that the temperature is correctly computed. This may also include a check to confirm that the heating device is correctly connected and what type of heating device is connected, such as reading information from the connected device, such as reading a RFID tag. Other conditions may be, that the laser unit is enabled, and a connection is verified. A loss in communication for a period of time, such as a period of 1.5sec to 7 sec, such as 1.5 sec, such as 5, sec, such as 7 sec, between the laser unit and the system or data incoherency may automatically stop the laser emission. In this way, the laser is turned off when the images are not updated and there is a risk that the user does not have near-real time information (loss of information and prolonged update rate can lead to unintentional tissue damage outside the treatment area which may be very bad when performing treatment of the brain and also in some other types of tissue.

Other mandatory conditions that may need to be fulfilled are baseline temperature is set and confirmed, such as core body temperature is confirmed;; drift compensation: reference baseline is set and confirmed; that a high temperature guard is provided, that there is a communication with the MR scanner, the test dose is successful and confirmed, etc.

S202: Test dose to confirm position of the heat providing area of the heating device, such as a laser-based heating device. This may be carried out by emitting a reduced power for a time, such as up to 30 sec. after the test dose has been confirmed, the power can be increased.

S203: Start emission, such as start laser emission. The emission may start at reduced power and is then automatically increased.

S204: Warming time: wait until the temperature has reached the target (43-60°C) in the specified region of interest used for controlling the treatment.

S205: "steady state" time: laser on/off automatic regulation based on the temperature measured by the specified region of interest used for controlling the treatment.

S206: Stop laser emission when treatment area achieved in damage map or automatically interrupted based on target damage, target treatment time reached or interrupted by guard temperature/damage reached or any other mandatory conditions no longer fulfilled.

S207: Sometimes it is possible to repeat steps S201 to S206 either by pulling back the fiber along the same track or by performing a new ablation in another track. For pull-back, the heating device may be configured to include a introduced which can be left with the heating device in tissue during treatment. The introduced may be transparent to the emission, such as transparent to the laser wavelength used. During the pull-back the heating device, such as a optical fiber, can be pulled back within the introducer which is left in its position. This provides the advantage that the risk of losing the position during the manipulation of the heating device during the pull-back is decreased. The heating device may also be protected at the same time. Alternatively, the introducer may be pulled-back too, thereby allowing the distal end of the heating device, such as the heat providing area which may be a diffusing portion of an optical fiber, to be in direct contact with the tissue.

Additionally and/or alternatively, as described for pull-back, an introducer may be configured to be left with the heating device in the tissue even if no pull-back should be performed.

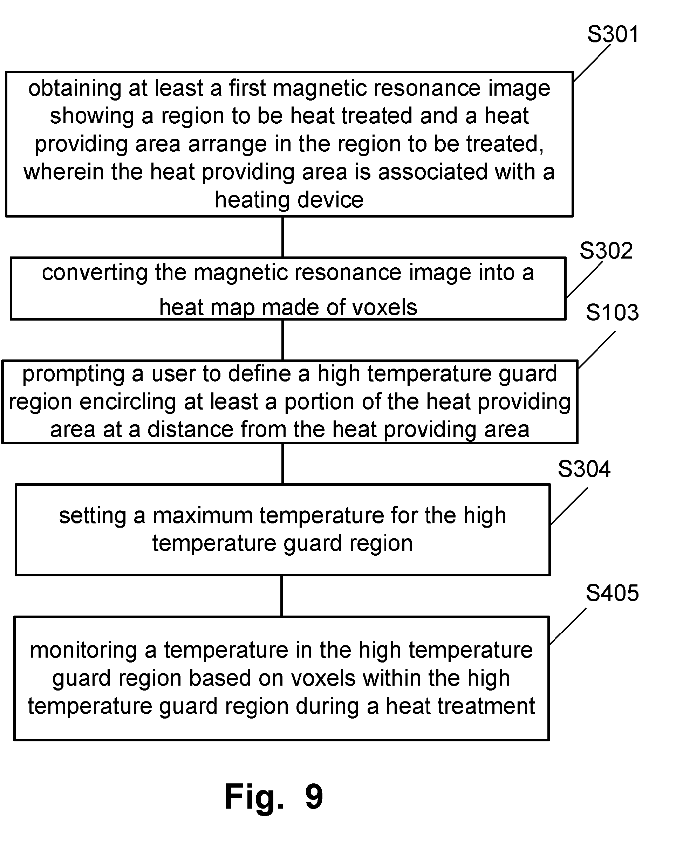

Fig. 9 is a schematic flow-chart illustrating a method for thermal therapy. The method may be implemented on a computer, such as a software. The method includes:

S301: obtaining at least a first magnetic resonance image showing a region to be heat treated and a heat providing area arrange in the region to be treated, wherein the heat providing area is associated with a heating device.

S302: converting the magnetic resonance image into a heat map made of voxels.

S303: prompting a user to define a high temperature guard region encircling at least a portion of the heat providing area at a distance from the heat providing area.

S304: setting a maximum temperature for the high temperature guard region.

S305 monitoring a temperature in the high temperature guard region based on voxels within the high temperature guard region during a heat treatment.

The method may further include a temperature monitoring guard is defined outside or inside the region of interest for feedback control of the thermal treatment, wherein the feedback control comprising:

- warming time: wait until the temperature has reached a target temperature at the temperature monitoring guard;
- regulating the heating device based on the temperature measured by the temperature monitoring guard;
- stop heat emission when achieved damage has been obtained based on target damage, target treatment time reached or interrupted by guard temperature/damage reached.

The method may further include that a region of interest covering the region to be heat treated is defined for monitoring a thermal damage. The region of interest may be the same area as the high temperature guard region. The thermal damage is based on thermal dose calculation, such as a thermal damage estimate, based on a temperature and exposure time of the tissue. Further, the region of interest may be divided into at least two different thermal damage thresholds (TDT) to display to display different levels of thermal damage. Additionally, in some examples, the method may stop heat emission when an achieved thermal damage has been obtained based on a target damage and/or a target treatment time reached.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

## Claims

1. A computer implemented method for thermal therapy, comprising:

   obtaining at least a first magnetic resonance image showing a region to be heat treated and a heat providing area arrange in the region to be treated, wherein the heat providing area is associated with a heating device,
   converting the magnetic resonance image into a heat map made of voxels;
   prompting a user to define a high temperature guard region encircling at least a portion of the heat providing area at a distance from the heat providing area;
   setting a maximum temperature for the high temperature guard region; and
   monitoring a temperature in the high temperature guard region based on voxels within the high temperature guard region during a heat treatment.

2. The computer implemented method of claim 1, switching off the heating device when the temperature reaches the maximum temperature; or reducing the power of the heating device when the temperature reaches the maximum temperature.

3. The computer implemented method of any of claims 1 to 2, wherein the heating device is a laser-based heat probe and the heat providing area is a light emitting area of the heat probe.

4. The computer implemented method of any of claims 1 to 3, receiving information from an interface device about the heating device connected and setting the maximum temperature based on the heating device connected.

5. The computer implemented method of any of claims 1 to 3, receiving information from an interface device about the heating device connected and setting a max power and a recommended power based on the heating device connected.

6. The computer implemented method of any of claims 2 to 5, wherein the maximum temperature is reached when at least one voxel, or a plurality of voxels, such as a defined number of voxels, inside the high temperature guard region is determined at the maximum temperature.

7. The computer implemented method of any of claims 1 to 6, wherein voxels within the high temperature guard region having a deviating temperature from neighboring voxels are discarded.

8. The computer implemented method of any of claims 1 to 7, wherein a region of interest covering the region to be heat treated is defined for monitoring a thermal damage .

9. The computer implemented method of claim 8, wherein the region of interest is the same as the high temperature guard region.

10. The computer implemented method of any of claims 8 to 9, wherein the thermal damage is based on thermal dose calculation, such as a thermal damage estimate, based on temperature and exposure time.

11. The computer implemented method of any of claims 8 to 10, wherein the region of interest is divided into at least two different thermal damage thresholds (TDT) to display to display different levels of thermal damage.

12. The computer implement method of any of claims 8 to 11, comprising stop heat emission when an achieved thermal damage has been obtained based on a target damage and/or a target treatment time reached.

13. The computer implemented method of any of claims 1 to 12, wherein a temperature monitoring guard is defined outside or inside the region to be treated, for feedback control of the thermal treatment, wherein the feedback control comprising:

    - warming time: wait until the temperature has reached a target temperature at the temperature monitoring guard;
    - regulating the heating device based on the temperature measured by the temperature monitoring guard.

14. The computer implemented method of any of claims 1 to 13, wherein a body temperature is estimated at a body temperature estimation area not affected by the thermal treatment and the heat map is corrected based on any changes to the body temperature estimation area.

15. The computer implemented method of any of claims 1 to 14, initiating a test dose at a power lower than the power for thermal treatment for a period, such as a predefined period of time, of time to confirm a position of the heat providing area and/or confirming a position of the at least a first magnetic resonance image, such as a thermometry plane, in relation to the heating provided area.

16. A system for thermal therapy comprising:

    a heating device comprising a heat providing area: and
a control unit configured to:

obtaining at least a first magnetic resonance image showing a region to be heat treated and the heat providing area arrange in the region to be treated,
converting the magnetic resonance image into a heat map made of voxels;
prompting a user to define a high temperature guard region encircling at least a portion of the heat providing area at a distance from the heat providing area;
setting a maximum temperature for the high temperature guard region;

and monitoring a temperature in the high temperature guard region based on voxels within the high temperature guard region during a heat treatment.

Fig. 1A

Fig. 1B

Fig. 2

**Fig. 3**

Fig. 4A

Fig. 4B

Fig. 4C

Fig. 5A

Fig. 5B

Fig. 5C

Fig. 5D

Fig. 6

S101

Fulfil all mandatory conditions

S102

Test dose to confirm heating device position

S103

Start emission and manual adjustment of power based on feedback from temperature and damage map

S104

Stop emission when treatment area achieved or automatically interrupted based on target damage, target treatment time reached or interrupted by guard temperature/damage reached or any other mandatory conditions no longer fulfilled

S105

Possible to repeat steps S101 to S104 either by pulling back the heatign device along the same track or by performing a new ablation in another track.

# Fig. 7

S201

Fulfil all mandatory conditions

S202

Test dose to confirm heating
device position

S203

Start emission

S204

Warming time: wait until the temperature has
reached the target in the specified ROI

S205

"steady state" time: Power to heating device on/off
automatic regulation based on the temperature
measured by the specified ROI

S206

Stop emission when treatment area achieved in
damage map or automatically interrupted based
on target damage, target treatment time reached
or interrupted by guard temperature/damage
reached or any other mandatory conditions no
longer fulfilled

S207

Possible to repeat steps S101 to S106 either by
pulling back the fiber along the same track or by
performing a new ablation in another track.

# Fig. 8

S301

obtaining at least a first magnetic resonance image showing a region to be heat treated and a heat providing area arrange in the region to be treated, wherein the heat providing area is associated with a heating device

S302

converting the magnetic resonance image into a heat map made of voxels

S103

prompting a user to define a high temperature guard region encircling at least a portion of the heat providing area at a distance from the heat providing area

S304

setting a maximum temperature for the high temperature guard region

S405

monitoring a temperature in the high temperature guard region based on voxels within the high temperature guard region during a heat treatment

Fig. 9

Europäisches
Patentamt
European
Patent Office
Office européen
des brevets

# EUROPEAN SEARCH REPORT

**Application Number**

EP 24 20 5429

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/000561 A1 (ZAGORCHEV LYUBOMIR GEORGIEV [US] ET AL) 5 January 2023 (2023-01-05) * paragraphs [0031], [0071], [0078], [0083]; figure 6A * | 1-16 | INV. A61B18/22 |
| X | US 2014/288542 A1 (TORCHIA MARK G [CA] ET AL) 25 September 2014 (2014-09-25) * paragraph [0149]; figures 1,13 * | 1-16 | |
| X | US 2016/051845 A1 (KOEHLER MAX OSKAR [NL]) 25 February 2016 (2016-02-25) | 1,2,4-16 | |
| A | * claim 1; figure 1 * | 3 | |
| X | US 2006/206105 A1 (CHOPRA RAJIV [CA] ET AL) 14 September 2006 (2006-09-14) | 1,2,4-16 | |
| A | * paragraphs [0041], [0054], [0067], [0075]; figures 1,2 * | 3 | |
| X | CN 107 822 707 A (UNIV SCIENCE & TECHNOLOGY CHINA) 23 March 2018 (2018-03-23) | 1,2,4-16 | |
| A | * the whole document * | 3 | TECHNICAL FIELDS SEARCHED (IPC) A61B |
| X | US 2023/142426 A1 (DYMLING STEPHAN [SE] ET AL) 11 May 2023 (2023-05-11) * paragraphs [0012], [0026], [0048] * | 1-16 | A61N A61F |
| X | US 2016/067519 A1 (TRANBERG KARL-GÖRAN [SE] ET AL) 10 March 2016 (2016-03-10) * paragraphs [0016], [0092], [0104]; figures 3A-C * | 1-16 | |

The present search report has been drawn up for all claims

| Place of search | Date of completion of the search | Examiner |
|---|---|---|
| Munich | 3 March 2025 | Link, Tatiana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 24 20 5429

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2025

| Patent document cited in search report | Publication date | Patent family member(s) | Publication date |
|---|---|---|---|
| US 2023000561 A1 | 05-01-2023 | US 2023000561 A1 | 05-01-2023 |
| | | WO 2023277996 A1 | 05-01-2023 |
| US 2014288542 A1 | 25-09-2014 | US 2008154252 A1 | 26-06-2008 |
| | | US 2013006230 A1 | 03-01-2013 |
| | | US 2014012241 A1 | 09-01-2014 |
| | | US 2014288542 A1 | 25-09-2014 |
| US 2016051845 A1 | 25-02-2016 | BR 112015026039 A2 | 25-07-2017 |
| | | CN 105208959 A | 30-12-2015 |
| | | EP 2986230 A1 | 24-02-2016 |
| | | JP 6353626 B2 | 04-07-2018 |
| | | JP 2016522696 A | 04-08-2016 |
| | | RU 2015149389 A | 24-05-2017 |
| | | US 2016051845 A1 | 25-02-2016 |
| | | WO 2014170138 A1 | 23-10-2014 |
| US 2006206105 A1 | 14-09-2006 | US 2006206105 A1 | 14-09-2006 |
| | | US 2011034833 A1 | 10-02-2011 |
| CN 107822707 A | 23-03-2018 | NONE | |
| US 2023142426 A1 | 11-05-2023 | AU 2017376750 A1 | 04-07-2019 |
| | | BR 112019012308 A2 | 12-11-2019 |
| | | CA 3046843 A1 | 21-06-2018 |
| | | CN 110191689 A | 30-08-2019 |
| | | CN 115500937 A | 23-12-2022 |
| | | EP 3335660 A1 | 20-06-2018 |
| | | EP 3554409 A1 | 23-10-2019 |
| | | EP 4169466 A1 | 26-04-2023 |
| | | ES 2865323 T3 | 15-10-2021 |
| | | ES 2939891 T3 | 27-04-2023 |
| | | IL 267261 A | 29-08-2019 |
| | | IL 296671 A | 01-11-2022 |
| | | JP 7231936 B2 | 02-03-2023 |
| | | JP 7525927 B2 | 31-07-2024 |
| | | JP 2020501686 A | 23-01-2020 |
| | | JP 2022188267 A | 20-12-2022 |
| | | JP 2024059940 A | 01-05-2024 |
| | | KR 20190107020 A | 18-09-2019 |
| | | US 2019357978 A1 | 28-11-2019 |
| | | US 2023142426 A1 | 11-05-2023 |
| | | WO 2018109139 A1 | 21-06-2018 |
| US 2016067519 A1 | 10-03-2016 | AU 2014261409 A1 | 12-11-2015 |
| | | BR 112015027594 A2 | 12-12-2017 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 24 20 5429

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2025

| Patent document cited in search report | Publication date | Patent family member(s) | Publication date |
|---|---|---|---|
| | | CN    105120948 A | 02-12-2015 |
| | | CN    109498152 A | 22-03-2019 |
| | | CN    109771030 A | 21-05-2019 |
| | | EP      2799111 A1 | 05-11-2014 |
| | | EP      2991730 A1 | 09-03-2016 |
| | | EP      3335762 A1 | 20-06-2018 |
| | | ES      2734518 T3 | 10-12-2019 |
| | | ES      2940352 T3 | 05-05-2023 |
| | | JP      6709153 B2 | 10-06-2020 |
| | | JP      7190463 B2 | 15-12-2022 |
| | | JP    2016522024 A | 28-07-2016 |
| | | JP    2020142111 A | 10-09-2020 |
| | | KR   20160026865 A | 09-03-2016 |
| | | US    2016067519 A1 | 10-03-2016 |
| | | US    2021236839 A1 | 05-08-2021 |
| | | US    2024082596 A1 | 14-03-2024 |
| | | WO    2014177663 A1 | 06-11-2014 |

EPO FORM P0459

For more details about this annex : see Official Journal of the European Patent Office, No. 12/82

page 2 of 2

## REFERENCES CITED IN THE DESCRIPTION

**Patent documents cited in the description**

- WO 2014177663 A1

**Non-patent literature cited in the description**

- **ISHIHARA, Y. et al.** A precise and fast temperature mapping using water proton chemical shift. *Magnetic Resonance in Medicine*, vol. 34 (6), 814-823

- **SAPARETO, SA** ; **DEWEY, WC**. Thermal dose determination in cancer therapy. *Int J Radiat Oncol Biol Phys.*, 1984, vol. 10, 787-800